# EUROPEAN PATENT APPLICATION

(11) **EP 1 728 424 A2**
(43) Date of publication of application: **06.12.2006**
(21) Application number: 06118442.0
(22) Date of filing: 11.12.2003
(51) Int. Cl.: A01K 67/033, C07K 14/435, C12Q 1/68, A61P 3/10

(54) **Method to treat conditions associated with insulin signaling dysregulation**

(30) Priority: 12.12.2002 US 432854 P
(62) Divisional of application: 03795884.0
(71) Applicant: Novartis AG, 4002 Basel (CH); Novartis Pharma GmbH, 1230 Vienna (AT)
(72) Inventor: Li, Hao, Warren, NJ 07959 (US)
(74) Representative: Crawley, Patrick Edward

(57) **Abstract**

The invention discloses a method to identify proteins involved in the insulin signaling pathway. The invention also discloses suitable targets for the development of new therapeutics to treat, prevent or ameliorate pathological conditions associated with dysregulation of the insulin signaling pathway. The invention also relates to methods to treat, prevent or ameliorate said conditions and pharmaceutical compositions therefor as well as to a method to identify compounds with therapeutic usefulness to treat pathological conditions associated with dysregulation of the insulin signaling pathway.

## Description

### FIELD OF THE INVENTION

This invention relates to a method to identify proteins involved in the insulin signaling pathway, methods for identifying compounds useful to treat pathologial conditions associated with dysregulation of the insulin signaling pathway as well as to methods and pharmaceutical compositions to treat, prevent or ameliorate conditions associated with dysregulation of the insulin signaling pathway.

### BACKGROUND OF THE INVENTION

The regulation of numerous cellular processes involves the transmission of extracelluar information through the cell via a network of associated proteins. Defects in signal transduction mechanisms may be associated with specific diseases, for example, Type II or noninsulin-dependent diabetes mellitus (NIDDM) is thought to be due largely due to defects in the insulin signaling pathway.

The molecular mechanisms of insulin signaling have been extensively reviewed. (Avruch, J. (1998).. Mol Cell Biochem 182, 31-48; Combettes-Souverain, M., and lssad, T. (1998). Diabetes Metab 24, 477-89; Kahn, B. B. (1998) Cell 92, 593-6; Virkamaki, A., et al. (1999). J Clin Invest 103, 931-43). Recent genetic studies in C. elegans and Drosophila have shown a remarkable conservation of the insulin pathways in invertebrates and mammals ( Paradis, S.et al. (1999) Genes Dev 13, 1438-52; Weinkove, D., and Leevers, S. J. (2000). Curr Opin Genet Dev 10, 75-80). In addition, many aspects of the insulin pathway are conserved between humans and *Drosophila.* For example, almost all currently known genes involved in the insulin signaling pathway in humans are also found in *Drosophila* and have similar biochemical functions.

Using Drosophila as a model system, Applicants herein disclose a method to identify proteins involved in the insulin signaling pathway. Employing said method, Applicants have discovered and describe herein several new proteins involved in the insulin signaling pathway. It is contemplated herein that these proteins and the genes encoding said proteins may serve as drug targets for the development of therapeutics to treat, prevent or ameliorate diabetes and other pathological conditions associated with dysregulation of the insulin signalling pathway.

### SUMMARY OF THE INVENTION

The instant application discloses a method to employ transgenic Drosophila to identify proteins involved in the insulin signaling pathway. Human homologs of the Drosophila genes identified according to this method are suitable targets for the development of new therapeutics to treat, prevent or ameliorate pathological conditions associated with the dysregulation of the insulin signaling pathway. Thus, in one aspect the invention relates to a method to identify modulators useful to treat or ameliorate said conditions, including Type II diabetes and the Type A syndrome of insulin resistance comprising a) assaying for the ability of a candidate modulator to modulate the biochemical function of a protein selected from the group consisting of those disclosed in Table 4 and/or modulate gene expression of said protein and which can further include b) assaying for the ability of an identified modulator to reverse the pathological effects observed in animal models of pathological conditions associated with the dysregulation of the insulin signaling pathway and/ or in clinical studies with subjects with said conditions.

In another aspect, the invention relates to a method to treat, prevent or ameliorate pathological conditions associated with the dysregulation of the insulin signaling pathway, including Type II diabetes and the Type A syndrome of insulin resistance, comprising administering to a subject in need thereof an effective amount of a modulator of a protein selected from the group consisting of those disclosed in Table 4, wherein said modulator, e.g., inhibits or enhances the biochemical function of said protein. In a further embodiment, the modulator comprises antibodies to said protein or fragments thereof, wherein said antibodies can inhibit the biochemical function of said protein in said subject.

In another embodiment the modulator inhibits or enhances the gene expression of a protein selected from the group consisting of those disclosed in Table 4. In a further embodiment, the modulator comprises any one or more substances selected from the group consisting of antisense oligonucleotides, triple helix DNA, ribozymes, RNA aptamers, siRNA and double or single stranded RNA wherein said substances are designed to inhibit gene expression of said protein.

In another aspect, the invention relates to a method to treat, prevent or ameliorate pathological conditions associated with dysregulation of the insulin signaling pathway, including Type II diabetes and the Type A syndrome of insulin resistance, comprising administering to a subject in need thereof a pharmaceutical composition comprising an effective amount of a modulator of a protein selected from the group consisting of those disclosed in Table 4.. In various embodiments, said pharmaceutical composition comprises antibodies to said protein or fragments thereof, wherein said antibodies can inhibit the biochemical function of said protein in said subject and/or any one or more substances selected from the group consisting of antisense oligonucleotides, triple helix DNA, ribozymes, RNA aptamers, siRNA and double or single stranded RNA wherein said substances are designed to inhibit gene expression of said protein.

In another aspect, the invention relates to a pharmaceutical composition comprising a modulator to a protein selected from the group consisting of those disclosed in Table 4 in an amount effective to treat, prevent or ameliorate a pathological condition associated with dysregulation of the insulin signaling pathway, including Type II diabetes and the Type A syndrome of insulin resistance, in a subject in need thereof. In one embodiment, said modulator may e.g., inhibit or enhance the biochemical functions of said protein. In a further embodiment said modulator comprises antibodies to said protein or fragments thereof, wherein said antibodies can, e.g., inhibit the biochemical functions of said protein.

In a further embodiment, said pharmaceutical composition comprises a modulator which may e.g., inhibit or enhance gene expression of said protein. In a further embodiment, said modulator comprises any one or more substances selected from the group consisting of antisense oligonucleotides, triple helix DNA, ribozymes, RNA aptamers, siRNA or double or single stranded RNA directed to a nucleic acid sequence of said protein wherein said substances are designed to inhibit gene expression of said protein.

In another aspect, the invention relates to a method to diagnose subjects suffering from pathological conditions associated with dysregulation of the insulin signaling pathway who may be suitable candidates for treatment with modulators to a protein selected from the group consisting of those disclosed in Table 4 comprising detecting levels of any one or more of said proteins in a biological sample from said subject wherein subjects with altered levels compared to controls would be suitable candidates for modulator treatment.

In another aspect, the invention relates to a method to diagnose subjects suffering from pathological conditions associated with dysregulation of the insulin signaling pathway who may be suitable candidates for treatment with modulators to a protein selected from the group consisting of those disclosed in Table 4 comprising assaying mRNA levels of any one or more of said protein in a biological sample from said subject wherein subjects with altered levels compared to controls would be suitable candidates for modulator treatment.

In yet another aspect, there is provided a method to treat, prevent or ameliorate pathological conditions associated with dysregulation of the insulin signaling pathway, including Type II diabetes and the Type A syndrome of insulin resistance comprising: (a) assaying for mRNA and/or protein levels of a protein selected from the group consisting of those disclosed in Table 4 in a subject; and (b) administering to a subject with altered levels of mRNA and/or protein levels compared to controls a modulator to said protein in an amount sufficient to treat, prevent or ameliorate the pathological effects of said condition. In particular embodiments, said modulator inhibits or enhances the biochemical function of said protein or gene expression of said protein.

In yet another aspect of the present invention there are provided assay methods and diagnostic kits comprising the components necessary to detect mRNA levels or protein levels of any one or more proteins selected from the group consisting of those disclosed in Table 4 in a biological sample, said kit comprising e.g. polynucleotides encoding any one or more proteins selected from the group consisting of those disclosed in Table 4; nucleotide sequences complementary to said protein; any one or more of said proteins, or fragments thereof of antibodies that bind to any one or more of said proteins, or to fragments thereof. In a preferred embodiment, such kits also comprise instructions detailing the procedures by which the kit components are to be used.

The present invention also pertains to the use of a modulator to a protein selected from the group consisting of those disclosed in Table 4 in the manufacture of a medicament for the treatment, prevention or amelioration of pathological conditions associated with dysregulation of the insulin signaling pathway, including Type II diabetes and the Type A syndrome of insulin resistance. In one embodiment, said modulator comprises any one or more substances selected from the group consisting of antisense oligonucleotides, triple helix DNA, ribozymes, RNA aptamer, siRNA and double or single stranded RNA wherein said substances are designed to inhibit gene expression of said protein. In yet a further embodiment, said modulator comprises one or more antibodies to said protein or fragments thereof, wherein said antibodies or fragments thereof can,. e.g., inhibit the biochemical function of said protein.

The invention also pertains to a modulator to a protein selected from the group consisting of those disclosed in Table 4 for use as a pharmaceutical. In one embodiment, said modulator comprises any one or more substances selected from the group consisting of antisense oligonucleotides, triple helix DNA, ribozymes, RNA aptamer, siRNA and double or single stranded RNA wherein said substances are designed to inhibit gene expression of said protein. In yet a further embodiment, said modulator comprises one or more antibodies to said protein or fragments thereof, wherein said antibodies or fragments thereof can, e.g., inhibit the biochemical functions of said protein.

In another aspect, the invention also pertains to a method to identify proteins involved in the insulin signaling pathway, said method comprising providing a transgenic fly whose genome comprises a DNA sequence encoding a polypeptide comprising the dominant negative PI3K catalytic subunit *Dp110*^{*D954A*}*,* said DNA sequence operably linked to a tissue specific expression control sequence, and expressing said DNA sequence, wherein expression of said DNA sequence results in said fly displaying a transgenic phenotype compared to controls; crossing said transgenic fly with a fly containing a mutation in a known or predicted gene; and screening progeny of said crosses for flies that carry said DNA sequence and said mutation and display modified expression of the transgenic phenotype as compared to appropriate controls. In one embodiment, said DNA sequence encodes *Dp110*^{*D954A*} and said tissue specific expression control sequence comprises the eye-specific enhancer ey-Gal4 and expression of said DNA sequence results in said fly displaying the "small eye" phenotype.

In a particular embodiment the invention relates to a method to identify drug targets for the development of therapeutics to treat, prevent or ameliorate pathological conditions associated with dysregulation of the insulin signaling pathway, including Type II diabetes and Type A syndrome of insulin resistance, said method comprising identifying the human homologs of the Drosophila proteins identified according to the method discussed above.

Other objects, features, advantages and aspects of the present invention will become apparent to those of skill from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### DETAILED DESCRIPTION OF THE INVENTION

All patent applications, patents and literature references cited herein are hereby incorporated by reference in their entirety.

Abbreviations used in the following description include:
IRSs, insulin receptor substrates.
PI3K, phosphoinositide 3-kinase.
PDKs, 3'-phosphoinositide-dependent protein kinases.
PTEN, phosphatase and tensin homolog deleted from chromosome 10.
PKB, protein kinase B, also known as Akt1.

In practicing the present invention, many conventional techniques in molecular biology, microbiology, and recombinant DNA are used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively). Well known Drosophila molecular genetics techniques can be found, for example, in Robert, D.B., Drosophila, A Practical Approach (IRL Press, Washington D.C. 1986).

Descriptions of flystocks can be found in the Flybase database at http://flybase.bio.indiana.edu.

Stock centers referred to herein include Bloomington and Szeged stock centers which are located at Bloomington, Indiana and Szeged, Hungary, respectively.

As used herein and in the appended claims, the singular forms "a", "an", and "the" include plural reference unless the context clearly dictates otherwise. Thus, for example, reference to "the antibody" is a reference to one or more antibodies and equivalents thereof known to those skilled in the art, and so forth.

"Nucleic acid sequence", as used herein, refers to an oligonucleotide, nucleotide or polynucleotide, and fragments or portions thereof, and to DNA or RNA of genomic or synthetic origin that may be single or double stranded, and represent the sense or antisense strand.

The term "antisense" as used herein, refers to nucleotide sequences which are complementary to a specific DNA or RNA sequence. The term "antisense strand" is used in reference to a nucleic acid strand that is complementary to the "sense" strand. Antisense molecules may be produced by any method, including synthesis by ligating the gene(s) of interest in a reverse orientation to a viral promoter which permits the synthesis of a complementary strand. Once introduced into a cell, this transcribed strand combines natural sequences produced by the cell to form duplexes. These duplexes then block either the further transcription or translation. The designation "negative" is sometimes used in reference to the antisense strand, and "positive" is sometimes used in reference to the sense strand.

"cDNA" refers to DNA that is complementary to a portion of messenger RNA (mRNA) sequence and is generally synthesized from an mRNA preparation using reverse transcriptase.

As contemplated herein, antisense oligonucleotides, triple helix DNA, RNA aptamers, ribozymes, siRNA and double or single stranded RNA are directed to a nucleic acid sequence such that the nucleotide sequence chosen will produce gene-specific inhibition of gene expression. For example, knowledge of a nucleotide sequence may be used to design an antisense molecule which gives strongest hybridization to the mRNA. Similarly, ribozymes can be synthesized to recognize specific nucleotide sequences of a gene and cleave it (Cech. J. Amer. Med Assn. 260:3030 (1988)). Techniques for the design of such molecules for use in targeted inhibition of gene expression is well known to one of skill in the art.

The individual proteins/polypeptides referred to herein include any and all forms of these proteins including, but not limited to, partial forms, isoforms, variants, precursor forms, the full length protein, fusion proteins containing the sequence or fragments of any of the above, from human or any other species. Protein homologs or orthologs which would be apparent to one of skill in the art are included in this definition. It is also contemplated that the term refers to proteins isolated from naturally occurring sources of any species such as genomic DNA libraries as well as genetically engineered host cells comprising expression systems, or produced by chemical synthesis using, for instance, automated peptide synthesizers or a combination of such methods. Means for isolating and preparing such polypeptides are well understood in the art.

The term "sample" as used herein, is used in its broadest sense. A biological sample from a subject may comprise blood, urine, brain tissue, primary cell lines, immortilized cell lines, or other biological material with which protein activity or gene expression may be assayed. A biological sample may include, for example, blood, tumors or other specimens from which total RNA may be purified for gene expression profiling using, for example, conventional glass chip microarray technologies such as Affymetrix chips, RT-PCR or other conventional methods.

As used herein, the term "antibody" refers to intact molecules as well as fragments thereof, such as Fa, F(ab')₂, and Fv, which are capable of binding the epitopic determinant. Antibodies that bind specific polypeptides can be prepared using intact polypeptides or fragments containing small peptides of interest as the immunizing antigen. The polypeptides or peptides used to immunize an animal can be derived from the translation of RNA or synthesized chemically, and can be conjugated to a carrier protein. Commonly used carriers that are chemically coupled to peptides include bovine serum albumin and thyroglobulin. The coupled peptide is then used to immunize an animal (e.g., a mouse, goat, chicken, rat or a rabbit).

The term "humanized antibody" as used herein, refers to antibody molecules in which amino acids have been replaced in the non-antigen binding regions in order to more closely resemble a human antibody, while still retaining the original binding ability.

A "therapeutically effective amount" is the amount of drug sufficient to treat, prevent or ameliorate pathological conditions associated with dysregulation of the insulin signaling pathway.

A "transgenic" organism as used herein refers to an organism that has had extra genetic material inserted into its genome. As used herein, a "transgenic fly" includes embryonic, larval and adult forms of *Drosophila* that contain a DNA sequence from the same or another organism randomly inserted into their genome. Although *Drosophila melanogaster* is preferred, it is contemplated that any fly of the genus *Drosophila* may be used in the present invention.

As used herein, "ectopic" expression of the transgene refers to expression of the transgene in a tissue or cell or at a specific developmental stage where it is not normally expressed.

As used herein, "phenotype" refers to the observable physical or biochemical characteristics of an organism as determined by both genetic makeup and environmental influences.

The term "transcription factor" refers to any protein required to inititate or regulate transcription in eukaryotes. For example, the eye-specific promoter GMR is a binding site for the eye-specific transcription factor, GLASS (Moses, K and Rubin, GM Genes Dev. 5(4):583-93 (1991)).

"UAS" region as used herein refers to an upstream activating sequence recognized by the GAL-4 transcriptional activator.

As used herein, a "control" fly refers to a larva or fly that is of the same genotype as larvae or flies used in the methods of the present invention except that the control larva or fly does not carry the mutation being tested for modification of phenotype.

As used herein, a "transformation vector" is a modified transposable element used with the transposable element technique to mediate integration of a piece of DNA in the genome of the organism and is familiar to one of skill in the art.

As used herein, "elevated transcription of mRNA" refers to a greater amount of messenger RNA transcribed from the natural endogenous gene encoding a protein, e.g. a human protein set forth in Table 4, compared to control levels. Elevated mRNA levels of a protein, e.g. a . human protein disclosed on Table 4, may be present in a tissue or cell of an individual suffering from a pathological condition associated with dysregulation of the insulin signaling pathway compared to levels in a subject not suffering from said condition. In particular, levels in a subject suffering from said condition may be at least about twice, preferably at least about five times, more preferably at least about ten times, most preferably at least about 100 times the amount of mRNA found in corresponding tissues in humans who do not suffer from said condition. Such elevated level of mRNA may eventually lead to increased levels of protein translated from such mRNA in an individual suffering from a pathological condition associated with dysregulation of the insulin signaling pathway as compared to levels in a healthy individual.

As used herein, a "Drosophila transformation vector" is a DNA plasmid that contains transposable element sequences and can mediate integration of a piece of DNA in the genome of the organism. This technology is familiar to one of skill in the art.

As used herein, the "small eye phenotype" is characterized by reduced cell size in the eye tissue compared to appropriate controls (Leevers, SJ et al. EMBO J. 1996 Dec 2; 15 (23):6584-94).

Methods of obtaining transgenic organisms, including transgenic *Drosophila,* are well known to one skilled in the art. For example, a commonly used reference for P-element mediated transformation is Spradling, 1986, "P element mediated transformation", In Drosophila: A practical approach (ed. D. B. Roberts), pp175-197, IRL Press, Oxford, UK. The EP element technology refers to a binary system, utilizing the yeast Gal4 transcriptional activator, that is used to ectopically regulate the transcription of endogenous Drosophila genes. This technology is described in Brand and Perrimon, 1993. "Targeted gene expression as a means of altering cell fates and generating dominant phenotypes", Development 118, pp401-415 and in : Rorth et al, 1998, "Systematic gain-of-function genetics in Drosophila" Development, 125(6), pp1049-1057.

A "host cell," as used herein, refers to a prokaryotic or eukaryotic cell that contains heterologous DNA that has been introduced into the cell by any means, e.g., electroporation, calcium phosphate precipitation, microinjection, transformation, viral infection, and the like.

"Heterologous" as used herein means "of different natural origin" or represent a non-natural state. For example, if a host cell is transformed with a DNA or gene derived from another organism, particularly from another species, that gene is heterologous with respect to that host cell and also with respect to descendants of the host cell which carry that gene. Similarly, heterologous refers to a nucleotide sequence derived from and inserted into the same natural, original cell type, but which is present in a non-natural state, e.g. a different copy number, or under the control of different regulatory elements.

A "vector" molecule is a nucleic acid molecule into which heterologous nucleic acid may be inserted which can then be introduced into an appropriate host cell. Vectors preferably have one or more origin of replication, and one or more site into which the recombinant DNA can be inserted. Vectors often have convenient means by which cells with vectors can be selected from those without, e.g., they encode drug resistance genes. Common vectors include plasmids, viral genomes, and (primarily in yeast and bacteria) "artificial chromosomes."

"Plasmids" generally are designated herein by a lower case p preceded and/or followed by capital letters and/or numbers, in accordance with standard naming conventions that are familiar to those of skill in the art. Starting plasmids disclosed herein are either commercially available, publicly available on an unrestricted basis, or can be constructed from available plasmids by routine application of well known, published procedures. Many plasmids and other cloning and expression vectors that can be used in accordance with the present invention are well known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated, even if subsequently reintroduced into the natural system. Such polynucleotides could be part of a vector and/or such polynucleotides or polypeptides could be part of a composition, and still be isolated in that such vector or composition is not part of its natural environment.

As used herein, the terms "transcriptional control sequence" or "expression control sequence" refer to DNA sequences, such as initiator sequences, enhancer sequences, and promoter sequences, which induce, repress, or otherwise control the transcription of a protein encoding nucleic acid sequences to which they are operably linked. They may be tissue specific and developmental- stage specific. A "human transcriptional control sequence" is a transcriptional control sequence normally found associated with the human gene encoding a polypeptide set forth in Table 4 of the present invention as it is found in the respective human chromosome. A "non-human transcriptional control sequence" is any transcriptional control sequence not found in the human genome.

The term "polypeptide" is used interchangeably herein with the terms "polypeptides" and "protein(s)".

A "chemical derivative" of a protein set forth in Table 4 of the invention is a polypeptide that contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed, for example, in Remington's Pharmaceutical Sciences, 16th ed., Mack Publishing Co., Easton, Pa. (1980).

The ability of a substance to "modulate" a protein set forth in Table 4 (i.e. "a modulator of a protein selected from the group consisting of those disclosed in Table 4") includes, but is not limited to, the ability of a substance to inhibit the activity of said protein and/or inhibit the gene expression of said protein. Such modulation could also involve effecting the ability of other proteins to interact with said protein, for example related regulatory proteins or proteins that are modified by said protein.

The term "agonist", as used herein, refers to a molecule (i.e. modulator) which, directly or indirectly, may modulate a polypeptide (e.g. a polypeptide set forth in Table 4) and which increase the biological activity of said polypeptide. Agonists may include proteins, nucleic acids, carbohydrates, or other molecules A modulator that enhances gene transcription or the biochemical function of a protein is something that increases transcription or stimulates the biochemical properties or activity of said protein, respectively.

The terms "antagonist" or "inhibitor" as used herein, refer to a molecule (i.e. modulator) which directly or indirectly may modulate a polypeptide (e.g. a polypeptide set forth in Table 4) which blocks or inhibits the biological activity of said polypeptide. Antagonists and inhibitors may include proteins, nucleic acids, carbohydrates, or other molecules. A modulator that inhibits gene expression or the biochemical function of a protein is something that reduces gene expression or biological activity of said protein, respectively.

As used herein, "pathological condition associated with dysregulation of the insulin signaling pathway" includes, but is not limited to, diabetes, e.g., Type II diabetes, gestational diabetes and the Type A syndrome of insulin resistance.

As generally referred to herein, a protein or gene selected from the group consisting of those disclosed in Table 4 refers to the human form of the gene.

"In vivo models of a pathological condition associated with dysregulation of the insulin signaling pathway" include those in vivo models of diabetes familiar to those of skill in the art. Such in vivo models include: an association of the common pro12 allele in PPAR-gamma with type-2 diabetes (Altshuler, D. et al., Nature Genet. 76-80, 2000), defects in the human insulin receptor gene (Kadowaki, T., et al. Science 240: 787-790, 1988), defects in the insulin receptor substrate 1 gene in mouse (Abe, H.; et al., J. Clin. Invest. 101: 1784-1788, 1998) and defects in glycogen sythase in humans (Groop, L. C et al., New Eng. J. Med. 328: 10-14, 1993).

The present invention discloses a method comprising using as a model organism a transgenic fly, *Drosophila melanogaster,* whose genome comprises a DNA sequence encoding the gene *Dp110*^{*D954A*}*.* Conventional expression control systems may be used to achieve ectopic expression of proteins of interest, including the *Dp110*^{*D954A*} peptide. Such expression may result in the disturbance of biochemical pathways and the generation of altered phenotypes. One such expression control system involves direct fusion of the DNA sequence to expression control sequences of tissue-specifically expressed genes, such as promoters or enhancers. A tissue specific expression control system that may be used is the binary Gal4-transcriptional activation system (Brand and Perrimon, Development 118:401-415 (1993)).

The Gal4 system uses the yeast transcriptional activator Gal4, to drive the expression of a gene of interest in a tissue specific manner. The Gal4 gene has been randomly inserted into the fly genome, using a conventional transformation system, so that it has come under the control of genomic enhancers that drive expression in a temporal and tissue-specific manner. Individual strains of flies have been established, called "drivers", that carry those insertions (Brand and Perrimon, Development 118:401-415 (1993)).

In the Gal4 system, a gene of interest is cloned into a transformation vector, so that its transcription is under the control of the *UAS* sequence (Upstream Activating Sequence), the Gal4-responsive element. When a fly strain that carries the *UAS*-gene of interest sequence is crossed to a fly strain that expresses the *Gal4* gene under the control of a tissue specific enhancer, the gene will be expressed in a tissue specific pattern.

In order to generate phenotypes that are easily visible in adult tissues and can thus be used in genetic screens, Gal4 "drivers" that drive expression in later stages of the fly development may be used in the present invention. Using these drivers, expression would result in possible defects in the wings, the eyes, the legs, different sensory organs and the brain. These "drivers" include, for example, *apterous-*Gal4 (wings) , elav-Gal4 (CNS), *sevenless*-Gal4, *eyeless*-Gal4 (also called ey-Gal4) and p*GMR*-Gal4 (eyes). Descriptions of the Gal4 lines and notes about their specific expression patterns is available in Flybase (http://flybase.bio.indiana.edu).

Various DNA constructs may be used to generate the transgenic *Drosophila melanogaster* disclosed herein. For example, the construct may contain the *Dp110*^{*D954A*} sequence cloned into the pUAST vector (Brand and Perrimon, Development 118:401-415 (1993)) which places the UAS sequence upstream of the transcribed region. Insertion of these constructs into the fly genome may occur through P-element recombination, Hobo element recombination (Blackman et al., EMBO J. 8:211-217 (1989)), homologous recombination (Rong and Golic, Science 288:2013-2018 (2000)) or other standard techniques known to one of skill in the art.

As discussed above, an ectopically expressed gene may result in an altered phenotype by disruption of a particular biochemical pathway. Mutations in genes acting in the same biochemical pathway are expected to cause modification of the altered phenotype. Thus, for example, a transgenic fly carrying both eyeless-Gal4 and UAS-- *Dp110*^{*D954A*} can be used to identify genes acting in the insulin signaling pathway by crossing this transgenic fly with a fly containing a mutation in a known or predicted gene; and screening progeny of the crosses for flies that display quantitative or qualitative modification of the altered phenotype of the eyeless-Gal4/*Dp110*^{*D954A*} transgenic fly, as compared to controls. Thus, this system is extremely beneficial for the elucidation of the function of *Dp110*^{*D954A*} products, as well as the identification of other genes that directly or indirectly interact with them. Mutations that can be screened include, but are not limited to, loss-of-function alleles of known genes, deletion strains, "enhancer-trap" strains generated by the P-element and gain-of-function mutations generated by random insertions into the Drosophila genome of a Gal4-inducible construct that can activate the ectopic expression of genes in the vicinity of its insertion. It is contemplated herein that genes involved in the insulin signaling pathway can be identified in this manner and these genes can then serve as targets for the development of therapeutics to treat pathological conditions associated with dysregulation in the insulin signaling pathway.

Nucleic acid molecules of the human homologs of the target polypeptides identifed according to the methods of the present invention and disclosed herein may act as target gene antisense molecules, useful, for example, in target gene regulation and/or as antisense primers in amplification reactions of target gene nucleic acid sequences. Further, such sequences may be used as part of ribozyme and/or triple helix sequences or as targets for siRNA or double or single stranded RNA, which may be employed for gene regulation. Still further, such molecules may be used as components of diagnostic kits as disclosed herein.

In cases where the gene identified using the methods of the present invention is the normal, or wild type, gene, this gene may be used to isolate mutant alleles of the gene. Such isolation is preferable in processes and disorders which are known or suspected to have a genetic basis. Mutant alleles may be isolated from individuals either known or suspected to have a genotype which contributes to disease symptoms related to pathological conditions associated with dysregulation of the insulin signaling pathway, including, but not limited to, conditions such as Type II diabetes or the Type A syndrome of insulin resistance. (Taylor, S.I and Ariogluo, E. (1998) J. Basic Clin. Physiol. Pharmacol. 9, 419-439). Mutant alleles and mutant allele products may then be utilized in the diagnostic assay systems described herein.

A cDNA of the mutant gene may be isolated, for example, by using PCR, a technique which is well known to those of skill in the art. In this case, the first cDNA strand may be synthesized by hybridizing an oligo-dT oligonucleotide to mRNA isolated from tissue known or suspected to be expressed in an individual putatively carrying the mutant allele, and by extending the new strand with reverse transcriptase. The second strand of the cDNA is then synthesized using an oligonucleotide that hybridizes specifically to the 5' end of the normal gene. Using these two primers, the product is then amplified via PCR, cloned into a suitable vector, and subjected to DNA sequence analysis through methods well known to those of skill in the art. By comparing the DNA sequence of the mutant gene to that of the normal gene, the mutation(s) responsible for the loss or alteration of function of the mutant gene product can be ascertained.

Alternatively, a genomic or cDNA library can be constructed and screened using DNA or RNA, respectively, from a tissue known to or suspected of expressing the gene of interest in an individual suspected of or known to carry the mutant allele. The normal gene or any suitable fragment thereof may then be labeled and used as a probe to identify the corresponding mutant allele in the library. The clone containing this gene may then be purified through methods routinely practiced in the art, and subjected to sequence analysis as described above.

Additionally, an expression library can be constructed utilizing DNA isolated from or cDNA synthesized from a tissue known to or suspected of expressing the gene of interest in an individual suspected of or known to carry the mutant allele. In this manner, gene products made by the putatively mutant tissue may be expressed and screened using standard antibody screening techniques in conjunction with antibodies raised against the normal gene product, as described below. (For screening techniques, see, for example, Harlow, E. and Lane, eds., 1988, "Antibodies: A Laboratory Manual", Cold Spring Harbor Press, Cold Spring Harbor). In cases where the mutation results in an expressed gene product with altered function (e.g., as a result of a missense mutation), a polyclonal set of antibodies are likely to cross-react with the mutant gene product. Library clones detected via their reaction with such labeled antibodies can be purified and subjected to sequence analysis as described above.

In another embodiment, nucleic acids comprising a sequence encoding a polypeptide set forth in Table 4 or functional derivative thereof, may be administered to promote normal biological function, for example, normal insulin mediated signal transduction, by way of gene therapy. Gene therapy refers to therapy performed by the administration of a nucleic acid to a subject. In this embodiment of the invention, the nucleic acid produces its encoded protein that mediates a therapeutic effect by promoting a normal insulin signaling pathway.

Any of the methods for gene therapy available in the art can be used according to the present invention. Exemplary methods are described below.

In a preferred aspect, the therapeutic comprises a nucleic acid for a Table 4 polypeptide that is part of an expression vector that expresses a Table 4 protein or fragment or chimeric protein thereof in a suitable host. In particular, such a nucleic acid has a promoter operably linked to the Table 4 protein coding region, said promoter being inducible or constitutive, and, optionally, tissue-specific. In another particular embodiment, a nucleic acid molecule is used in which the Table 4 protein coding sequences and any other desired sequences are flanked by regions that promote homologous recombination at a desired site in the genome, thus providing for intrachromosomal expression of the Table 4 nucleic acid (Koller and Smithies, 1989, Proc. Natl. Acad. Sci. USA 86:8932-8935; Zijlstra et al., 1989, Nature 342:435-438).

Delivery of the nucleic acid into a patient may be either direct, in which case the patient is directly exposed to the nucleic acid or nucleic acid-carrying vector, or indirect, in which case, cells are first transformed with the nucleic acid in vitro, then transplanted into the patient. These two approaches are known, respectively, as *in vivo* or *ex vivo* gene therapy.

In a specific embodiment, the nucleic acid is directly administered *in vivo,* where it is expressed to produce the encoded product. This can be accomplished by any of numerous methods known in the art, e.g., by constructing it as part of an appropriate nucleic acid expression vector and administering it so that it becomes intracellular, e.g., by infection using a defective or attenuated retroviral or other viral vector (see, e.g., U.S. Pat. No. 4,980,286 and others mentioned *infra*), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see e.g., U.S. Patents 5,166,320; 5,728,399; 5,874,297; and 6,030,954, all of which are incorporated by reference herein in their entirety) (which can be used to target cell types specifically expressing the receptors), etc. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO92/20316; WO93/14188; and WO 93/20221). Alternatively, the nucleic acid can be introduced intracellularly and incorporated within host cell DNA for expression, by homologous recombination (see, e.g., U.S. Patents 5,413,923; 5,416,260; and 5,574,205; and Zijlstra et al., 1989, Nature 342:435-438).

In a specific embodiment, a viral vector that contains a nucleic acid encoding a Table 4 polypeptide is used. For example, a retroviral vector can be used (see, e.g., U.S. Patents 5,219,740; 5,604,090; and 5,834,182). These retroviral vectors have been modified to delete retroviral sequences that are not necessary for packaging of the viral genome and integration into host cell DNA. The nucleic acid for the Table 4 polypeptide to be used in gene therapy is cloned into the vector, which facilitates delivery of the gene into a patient.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Methods for conducting adenovirus-based gene therapy are described in, e.g., U.S. Patents 5,824,544; 5,868,040; 5,871,722; 5,880,102; 5,882,877; 5,885,808; 5,932,210; 5,981,225; 5,994,106; 5,994,132; 5,994,134; 6,001,557; and 6,033,8843, all of which are incorporated by reference herein in their entirety.

Adeno-associated virus (AAV) has also been proposed for use in gene therapy. Methods for producing and utilizing AAV are described, e.g., in U.S. Patents 5,173,414; 5,252,479; 5,552,311; 5,658,785; 5,763,416; 5,773,289; 5,843,742; 5,869,040; 5,942,496; and 5,948,675, all of which are incorporated by reference herein in their entirety.

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

The resulting recombinant cells can be delivered to a patient by various methods known in the art. In a preferred embodiment, epithelial cells are injected, e.g., subcutaneously. In another embodiment, recombinant skin cells may be applied as a skin graft onto the patient. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, the nucleic acid of a polypeptide set forth in Table 4 is introduced into the cells such that it is expressible by the cells or their progeny, and the recombinant cells are then administered in vivo for therapeutic effect. In a specific embodiment, stem or progenitor cells are used. Any stem-and/or progenitor cells that can be isolated and maintained in vitro can potentially be used in accordance with this embodiment of the present invention. Such stem cells include but are not limited to hematopoietic stem cells (HSC), stem cells of epithelial tissues such as the skin and the lining of the gut, embryonic heart muscle cells, liver stem cells (see, e.g., WO 94/08598), and neural stem cells (Stemple and Anderson, 1992, Cell 71:973-985).

Epithelial stem cells (ESCs) or keratinocytes can be obtained from tissues such as the skin and the lining of the gut by known procedures (Rheinwald, 1980, Meth. Cell Bio. 21A:229). In stratified epithelial tissue such as the skin, renewal occurs by mitosis of stem cells within the germinal layer, the layer closest to the basal lamina. Stem cells within the lining of the gut provide for a rapid renewal rate of this tissue. ESCs or keratinocytes obtained from the skin or lining of the gut of a patient or donor can be grown in tissue culture (Pittelkow and Scott, 1986, Mayo Clinic Proc. 61:771). If the ESCs are provided by a donor, a method for suppression of host versus graft reactivity (e.g., irradiation, drug or antibody administration to promote moderate immunosuppression) can also be used.

With respect to hematopoietic stem cells (HSC), any technique which provides for the isolation, propagation, and maintenance in vitro of HSC can be used in this embodiment of the invention. Techniques by which this may be accomplished include (a) the isolation and establishment of HSC cultures from bone marrow cells isolated from the future host, or a donor, or (b) the use of previously established long-term HSC cultures, which may be allogeneic or xenogeneic. Non-autologous HSC are used preferably in conjunction with a method of suppressing transplantation immune reactions of the future host/patient. In a particular embodiment of the present invention, human bone marrow cells can be obtained from the posterior iliac crest by needle aspiration (see, e.g., Kodo et al., 1984, J. Clin. Invest. 73:1377-1384). In a preferred embodiment of the present invention, the HSCs can be made highly enriched or in substantially pure form. This enrichment can be accomplished before, during, or after long-term culturing, and can be done by any techniques known in the art. Long-term cultures of bone marrow cells can be established and maintained by using, for example, modified Dexter cell culture techniques (Dexter et al., 1977, J. Cell Physiol. 91:335) or Witlock-Witte culture techniques (Witlock and Witte, 1982, Proc. Natl. Acad. Sci. USA 79:3608-3612).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

A further embodiment of the present invention relates to a method to treat, prevent or ameliorate a pathological condition associated with dysregulation of the insulin signaling pathway that comprises adminstering to a subject in need thereof an effective amount of a modulator of a protein selected from the group consisting of those disclosed in Table 4. In one embodiment, the modulator comprises one or more antibodies to said protein, or fragments thereof, wherein said antibodies or fragments thereof can inhibit the biochemical function of said protein in said subject

Described herein are methods for the production of antibodies capable of specifically recognizing one or more differentially expressed gene epitopes. Such antibodies may include, but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above. Such antibodies may be used, for example, in the detection of a target protein in a biological sample, or alternatively, as a method for the inhibition of the biochemical function of the protein. Thus, such antibodies may be utilized as part of disease treatment methods, and/or may be used as part of diagnostic techniques whereby patients may be tested e.g., for abnormal levels of polypeptides set forth in Table 4, or for the presence of abnormal forms of these polypeptides.

For the production of antibodies to the Table 4 polypeptides, various host animals may be immunized by injection with these polypeptides, or a portion thereof. Such host animals may include but are not limited to rabbits, mice, goats, chickens and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

Polyclonal antibodies are heterogeneous populations of antibody molecules derived from the sera of animals immunized with an antigen, such as target gene product, or an antigenic functional derivative thereof. For the production of polyclonal antibodies, host animals such as those described above, may be immunized by injection with a Table 4 polypeptide, or a portion thereof, supplemented with adjuvants as also described above.

Monoclonal antibodies, which are homogeneous populations of antibodies to a particular antigen, may be obtained by any technique that provides for the production of antibody molecules by continuous cell lines in culture. These include, but are not limited to the hybridoma technique of Kohler and Milstein, (1975, Nature 256:495-497; and U.S. Pat. No. 4,376,110), the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable or hypervariable region derived from a murine mAb and a human immunoglobulin constant region.

Alternatively, techniques described for the production of single chain antibodies (U.S. Pat. No. 4,946,778; Bird, 1988, Science 242:423-426; Huston et al., 1988, Proc. Natl. Acad. Sci. USA 85:5879-5883; and Ward et al., 1989, Nature 334:544-546) can be adapted to produce differentially expressed gene-single chain antibodies. Single chain antibodies are formed by linking the heavy and light chain fragments of the Fv region via an amino acid bridge, resulting in a single chain polypeptide.

Most preferably, techniques useful for the production of "humanized antibodies" can be adapted to produce antibodies to the polypeptides, fragments, derivatives, and functional equivalents disclosed herein. Such techniques are disclosed in U.S. Patent Nos. 5,932, 448; 5,693,762; 5,693,761; 5,585,089; 5,530,101; 5,910,771; 5,569,825; 5,625,126; 5,633,425; 5,789,650; 5,545,580; 5,661,016; and 5,770,429, the disclosures of all of which are incorporated by reference herein in their entirety.

Antibody fragments that recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

As contemplated herein, an antibody of the present invention can be preferably used in a diagnostic kit for detecting levels of a protein disclosed in Table 4 in a biological sample as well as in a method to diagnose subjects suffering from pathological conditions associated with dysregulation of the insulin signaling pathway who may be suitable candidates for treatment with modulators to a protein selected from the group consisting of those disclosed in Table 4. Preferably, said detecting step comprises contacting said appropriate tissue cell (e.g. biological sample) with an antibody which specifically binds to a Table 4 polypeptide or a fragment thereof and detecting specific binding of said antibody with a polypeptide in said appropriate tissue, cell or sample wherein detection of specific binding to a polypeptide indicates the presence of a polypeptide set forth in Table 4 or a fragment thereof.

Particularly preferred, for ease of detection, is the sandwich assay, of which a number of variations exist, all of which are intended to be encompassed by the present invention. For example, in a typical forward assay, unlabeled antibody is immobilized on a solid substrate and the sample to be tested brought into contact with the bound molecule. After a suitable period of incubation, for a period of time sufficient to allow formation of an antibody-antigen binary complex. At this point, a second antibody, labeled with a reporter molecule capable of inducing a detectable signal, is then added and incubated, allowing time sufficient for the formation of a ternary complex of antibody-antigen-labeled antibody. Any unreacted material is washed away, and the presence of the antigen is determined by observation of a signal, or may be quantitated by comparing with a control sample containing known amounts of antigen. Variations on the forward assay include the simultaneous assay, in which both sample and antibody are added simultaneously to the bound antibody, or a reverse assay in which the labeled antibody and sample to be tested are first combined, incubated and added to the unlabeled surface bound antibody. These techniques are well known to those skilled in the art, and the possibility of minor variations will be readily apparent. As used herein, "sandwich assay" is intended to encompass all variations on the basic two-site technique. For the immunoassays of the present invention, the only limiting factor is that the labeled antibody be an antibody which is specific for a Table 4 polypeptide or a fragment thereof.

The most commonly used reporter molecules in this type of assay are either enzymes, fluorophore- or radionuclide-containing molecules. In the case of an enzyme immunoassay, an enzyme is conjugated to the second antibody, usually by means of glutaraldehyde or periodate. As will be readily recognized, however, a wide variety of different ligation techniques exist, which are well-known to the skilled artisan. Commonly used enzymes include horseradish peroxidase, glucose oxidase, beta-galactosidase and alkaline phosphatase, among others. The substrates to be used with the specific enzymes are generally chosen for the production, upon hydrolysis by the corresponding enzyme, of a detectable color change. For example, p-nitrophenyl phosphate is suitable for use with alkaline phosphatase conjugates; for peroxidase conjugates, 1,2-phenylenediamine or toluidine are commonly used. It is also possible to employ fluorogenic substrates, which yield a fluorescent product rather than the chromogenic substrates noted above. A solution containing the appropriate substrate is then added to the tertiary complex. The substrate reacts with the enzyme linked to the second antibody, giving a qualitative visual signal, which may be further quantitated, usually spectrophotometrically, to give an evaluation of the amount of Table 4 polypeptide which is present in the serum sample.

Alternately, fluorescent compounds, such as fluorescein and rhodamine, may be chemically coupled to antibodies without altering their binding capacity. When activated by illumination with light of a particular wavelength, the fluorochrome-labeled antibody absorbs the light energy, inducing a state of excitability in the molecule, followed by emission of the light at a characteristic longer wavelength. The emission appears as a characteristic color visually detectable with a light microscope. Immunofluorescence and EIA techniques are both very well established in the art and are particularly preferred for the present method. However, other reporter molecules, such as radioisotopes, chemiluminescent or bioluminescent molecules may also be employed. It will be readily apparent to the skilled artisan how to vary the procedure to suit the required use.

The pharmaceutical compositions of the present invention may also comprise substances that inhibit the expression of a protein disclosed in Table 4 at the nucleic acid level. Such molecules include ribozymes, antisense oligonucleotides, triple helix DNA, RNA aptamers, siRNA and/or double or single stranded RNA directed to an appropriate nucleotide sequence of nucleic acid encoding such a protein. These inhibitory molecules may be created using conventional techniques by one of skill in the art without undue burden or experimentation. For example, modifications (e.g. inhibition) of gene expression can be obtained by designing antisense molecules, DNA or RNA, to the control regions of the genes encoding the polypeptides discussed herein, i.e. to promoters, enhancers, and introns. For example, oligonucleotides derived from the transcription initiation site, e.g., between positions - 10 and +10 from the start site may be used. Notwithstanding, all regions of the gene may be used to design an antisense molecule in order to create those which gives strongest hybridization to the mRNA and such suitable antisense oligonucleotides may be produced and identified by standard assay procedures familiar to one of skill in the art.

Similarly, inhibition of gene expression may be achieved using "triple helix" base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature (Gee, J.E. et al. (1994) In: Huber, B.E. and B. I. Carr, Molecular and Immunologic Approaches, Futura Publishing Co., Mt. Kisco, N.Y.). These molecules may also be designed to block translation of mRNA by preventing the transcript from binding to ribosomes.

Ribozymes, enzymatic RNA molecules, may also be used to inhibit gene expression by catalyzing the specific cleavage of RNA. The mechanism of ribozyme action involves sequence-specific hybridization of the ribozyme molecule to complementary target RNA, followed by endonucleolytic cleavage. Examples which may be used include engineered "hammerhead" or "hairpin" motif ribozyme molecules that can be designed to specifically and efficiently catalyze endonucleolytic cleavage of gene sequences. Specific ribozyme cleavage sites within any potential RNA target are initially identified by scanning the target molecule for ribozyme cleavage sites which include the following sequences: GUA, GUU and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Ribozyme methods include exposing a cell to ribozymes or inducing expression in a cell of such small RNA ribozyme molecules (Grassi and Marini, 1996, Annals of Medicine 28: 499-510; Gibson, 1996, Cancer and Metastasis Reviews 15: 287-299). Intracellular expression of hammerhead and hairpin ribozymes targeted to mRNA corresponding to at least one of the genes discussed herein can be utilized to inhibit protein encoded by the gene.

Ribozymes can either be delivered directly to cells, in the form of RNA oligonucleotides incorporating ribozyme sequences, or introduced into the cell as an expression vector encoding the desired ribozymal RNA. Ribozymes can be routinely expressed *in vivo* in sufficient number to be catalytically effective in cleaving mRNA, and thereby modifying mRNA abundance in a cell (Cotten et al., 1989 EMBO J. 8:3861-3866). In particular, a ribozyme coding DNA sequence, designed according to conventional, well known rules and synthesized, for example, by standard phosphoramidite chemistry, can be ligated into a restriction enzyme site in the anticodon stem and loop of a gene encoding a tRNA, which can then be transformed into and expressed in a cell of interest by methods routine in the art. Preferably, an inducible promoter (e.g., a glucocorticoid or a tetracycline response element) is also introduced into this construct so that ribozyme expression can be selectively controlled. For saturating use, a highly and constituently active promoter can be used. tDNA genes (i.e., genes encoding tRNAs) are useful in this application because of their small size, high rate of transcription, and ubiquitous expression in different kinds of tissues.

Therefore, ribozymes can be routinely designed to cleave virtually any mRNA sequence, and a cell can be routinely transformed with DNA coding for such ribozyme sequences such that a controllable and catalytically effective amount of the ribozyme is expressed. Accordingly, the abundance of virtually any RNA species in a cell can be modified or perturbed.

Ribozyme sequences can be modified in essentially the same manner as described for antisense nucleotides, e.g., the ribozyme sequence can comprise a modified base moiety.

RNA aptamers can also be introduced into or expressed in a cell to modify RNA abundance or activity. RNA aptamers are specific RNA ligands for proteins, such as for Tat and Rev RNA (Good et al., 1997, Gene Therapy 4: 45-54) that can specifically inhibit their translation.

Gene specific inhibition of gene expression may also be achieved using conventional double or single stranded RNA technologies. A description of such technology may be found in WO 99/32619 which is hereby incorporated by reference in its entirety. In addition, siRNA technology has also proven useful as a means to inhibit gene expression (Cullen, BR Nat. Immunol. 2002 Jul;3(7):597-9, Martinez, J. et al. Cell 2002 Sept.6;110(5):563).

Antisense molecules, triple helix DNA, RNA aptamers, dsRNA, ssRNA, siRNA and ribozymes of the present invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding the genes of the polypeptides discussed herein. Such DNA sequences may be incorporated into a wide variety of vectors with suitable RNA polymerase promoters such as T7 or SP6. Alternatively, cDNA constructs that synthesize antisense RNA constitutively or inducibly can be introduced into cell lines, cells, or tissues.

Vectors may be introduced into cells or tissues by many available means, and may be used in vivo, in vitro or ex vivo. For ex vivo therapy, vectors may be introduced into stem cells taken from the patient and clonally propagated for autologous transplant back into that same patient. Delivery by transfection and by liposome injections may be achieved using methods that are well known in the art.

Detection of mRNA levels of proteins disclosed herein may comprise contacting a biological sample or even contacting an isolated RNA or DNA molecule derived from a biological sample with an isolated nucleotide sequence of at least about 20 nucleotides in length that hybridizes under high stringency conditions (e.g. 0.1 x SSPE or SSC, 0.1 % SDS, 65°C) with the isolated nucleotide sequence encoding a polypeptide set forth in Table 4. Hybridization conditions may be highly stringent or less highly stringent. In instances wherein the nucleic acid molecules are deoxyoligonucleotides ("oligos"), highly stringent conditions may refer, e.g., to washing in 6X SSC/0.05% sodium pyrophosphate at 37 °C (for 14-base oligos), 48 °C (for 17-base oligos), 55 °C (for 20-base oligos), and 60 °C (for 23-base oligos). Suitable ranges of such stringency conditions for nucleic acids of varying compositions are described in Krause and Aaronson (1991), Methods in Enzymology, 200:546-556 in addition to Maniatis et al., cited above.

In some cases, detection of a mutated form of the gene which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

Nucleic acids, in particular mRNA, for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled nucleotide sequences encoding a polypeptide encoded by a gene disclosed in Table 4.. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing (e.g., Myers et al., Science (1985) 230:1242). Sequence changes at specific locations may also be revealed by nuclease protection assays, such as RNase and S1 protection or the chemical cleavage method (see Cotton et al., Proc Natl Acad Sci USA (1985) 85: 4397-4401). In addition, an array of oligonucleotides probes comprising nucleotide sequence encoding the Table 4 polypeptides or fragments of such nucleotide sequences can be constructed to conduct efficient screening of e.g., genetic mutations. Array technology methods are well known and have general applicability and can be used to address a variety of questions in molecular genetics including gene expression, genetic linkage, and genetic variability (see for example: M. Chee et al., Science, Vol 274, pp 610-613 (1996)).

The diagnostic assays offer a process for diagnosing or determining a susceptibility to disease through detection of mutation in the gene of a polypeptide set forth in Table 4 by the methods described. In addition, such diseases may be diagnosed by methods comprising determining from a sample derived from a subject an abnormally decreased or increased level of polypeptide or mRNA. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention relates to a diagnostic kit for detecting mRNA levels (or protein levels) which comprises:
(a) a polynucleotide of a polypeptide set forth in Table 4 or a fragment thereof;
(b) a nucleotide sequence complementary to that of (a);
(c) a polypeptide of Table 4 of the present invention encoded by the polynucleotide of (a),
(d) an antibody to the polypeptide of (c)
(e) an RNAi sequence complementary to that of (a)
It will be appreciated that in any such kit, (a), (b), (c), (d) or (e) may comprise a substantial component. Such a kit will be of use in diagnosing a disease or susceptibility to a disease, particularly to a disease or condition associated with dysregulation of the insulin signaling pathway, for example, Type II diabetes or the Type A syndrome of insulin resistance.

The nucleotide sequences of the present invention are also valuable for chromosome localization. The sequence is specifically targeted to, and can hybridize with, a particular location on an individual human chromosome. The mapping of relevant sequences to chromosomes is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

An additional embodiment of the invention relates to the administration of a pharmaceutical composition, in conjunction with a pharmaceutically acceptable carrier, excipient or diluent, for any of the therapeutic effects discussed above. Such pharmaceutical compositions may comprise, for example, a polypeptide set forth in Table 4, antibodies to that polypeptide, mimetics, agonists, antagonists, inhibitors or other modulators of function of a Table 4 polypeptide or gene therefore. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones.

In addition, any of the therapeutic proteins, antagonists, antibodies, agonists,antisense sequences or other modulators described above may be administered in combination with other appropriate therapeutic agents. Selection of the appropriate agents for use in combination therapy may be made by one of ordinary skill in the art, according to conventional pharmaceutical principles. The combination of therapeutic agents may act synergistically to effect the treatment, prevention or amelioration of pathological conditions associated with abnormalities in the insulin signaling pathway. Using this approach, one may be able to achieve therapeutic efficacy with lower dosages of each agent, thus reducing the potential for adverse side effects. Antagonists, agonists and other modulators of the human polypeptides set forth in Table 4 and genes encoding said polypeptides may be made using methods which are generally known in the art.

The pharmaceutical compositions encompassed by the invention may be administered by any number of routes including, but not limited to, oral, intravenous, intramuscular, intra-articular, intra-arterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations that can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated m aqueous solutions, preferably in physiologically compatible buffers such as Hanks' solution, Ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder that may contain any or all of the following: 1-50 mM histidine, 0.1%-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. Such labeling would include amount, frequency, and method of administration.

Pharmaceutical compositions suitable for use in the invention include compositions wherein the active ingredients are contained in an effective amount to achieve the intended purpose. The determination of an effective dose is well within the capability of those skilled in the art.

For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

A therapeutically effective dose refers to that amount of active ingredient which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutic effects is the therapeutic index, and it can be expressed as the ratio, LD50/ED50. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors that may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. Pharmaceutical formulations suitable for oral administration of proteins are described, e.g., in U.S. Patents 5,008,114; 5,505,962; 5,641,515; 5,681,811; 5,700,486; 5,766,633; 5,792,451; 5,853,748; 5,972,387; 5,976,569; and 6,051,561.

The following Examples illustrate the present invention, without in any way limiting the scope thereof.

### EXAMPLES

The following methods are employed to perform the examples provided below:

### Fly culture

All flies *(Drosophila melanogaster,* Bloomington Stock center and Szeged Stock center) are kept on standard corn meal food according to methods familiar to one of skill in the art. Using conventional methods, all crosses are done at 25°C. Balancers, or special chromosomes that prevent recombination and carry at least one dominant morphological marker are used and include *CyO, TM3* and *TM6B.* All flies used carry the *w*^{*1118*} mutant allele at the *white* locus. Mutations not referenced herein and the nomenclatures of standard *Drosophila* genetics have been described previously and are familiar to one of skill in the art (Lindsley, D. L., and Zimm, G. (1992). The Genome of Drosophila melanogaster (New York: Academic Press); http://flybase.bio.indiana.edu).

### Fly stocks

The *ey-Gal4* (also referred to as "eyeless-Gal4") strain was obtained from Bloomington stock center (Bloomington, IL). In this strain, the yeast transcription factor Gal4 is expressed in the precursor cells and adult cells of fly eyes. *UAS-Dp110*^{*D954A*} and *UAS-PKB* strains are used (Leevers et al, EMBO J. 1996 Dec 2;15(23):6584-94; Staveley, et al. Curr Biol. 1998 May 7;8(10):599-602). EP insertion strains were obtained from Bloomington and Szeged stock centers.

### Fly genetic crosses

The generation of a recombinant second chromosome that bears both the *ey-Gal4* and *UAS- Dp110*^{*D954A*} transgenes is described below: the *w; ey-Gal4*/*CyO* flies are crossed to *y w; UAS-Dp110*^{*D954A*} flies. Virgin daughters of the genotype *w*/*y w; ey-Gal4*/ *UAS- Dp110*^{*D954A*} are crossed to *w*/Y*; Sp*/*CyO; MKRS,ry*/*TM2, ry* males. A male progeny with the genotype *w*/Y*; ey-Gal4, UAS-Dp110*^{*D954A*}/*CyO; MKRS,ry*/*TM2, ry*/*+* is selected based on small eye phenotype and crossed to *w*/*w; Sp*/*CyO; MKRS, ry*/*TM2, ry* virgin females to establish *w; ey-Gal4, UAS-Dp110*^{*D954A*} /*CyO; MKRS,ry*/*TM2, ry*/*+* stock.

### Crosses using the X chromosome EP insertion lines

Crosses used for the screening of the X chromosome EP insertion lines are described below: Males of the X chromosome EP insertion lines (EP(X)) are crossed to *w; ey-Gal4, UAS-Dp110*^{*D954A*}/*CyO; MKRS,ry*/*TM2, ry*/*+* females. Adult progenies of the genotype EP(X)/*w; ey-Gal4 UAS-Dp110*^{*D954A*}/*+* and *wl*Y; *ey-GaI4, UAS-Dp110*^{*D954A*}/*+* are selected for comparison.

Flies are examined under a Zeiss dissecting microscope (Carl Zeiss Microlmaging, Inc. , Thornwood, NY). The eyes of at least five EP(X)/*w; ey-Gal4 UAS-Dp110*^{*D954A*}/*+* flies were compared to the eyes of at least five *w*/Y; *ey-Gal4, UAS-Dp110*^{*D954A*}/*+* (see above). If the average eye sizes of the two fly groups are significantly different upon visual inspection, the EP(X) line is selected as a potential positive. Statistical analysis is then performed by measuring the eye size of larger number of flies (see below).

### Crosses using the second chromosome EP insertion lines

The crosses used for the screening of the second chromosome EP insertion lines is described below: Either males or virgin females of the second chromosome EP insertion lines (EP(2)) are crossed to *w; ey-Gal4, UAS-Dp110*^{*D954A*}/*CyO; MKRS,ry*/*TM2, ryl+* virgin females or males. The eyes of at least five *ey-Gal4, UAS-Dp110*^{*D954A*}/*CyO* progeny flies are compared to the eyes of at least 5 *ey-Gal4, UAS-Dp110*^{*D954A*}/EP(2) progeny flies with the same sex. The same criterion was used to select potential positive EP(2) lines as in the case of EP(X) lines. Statistical analysis is also used to confirm the positives.

### Crosses using the third chromosome EP insertion lines

The crosses used for the screening of the third chromosome EP insertion lines is described below: The EP(3) lines obtained from stock centers are balanced either by TM3 or TM6B balancers. Either males or virgin females of the third chromosome EP insertion lines (EP(3)) are crossed to *w; ey-Gal4, UAS-Dp110*^{*D954A*} /*CyO; MKRS,ry*/*TM2, ry* /*+* virgin females or males. The eyes of at least five *ey-Gal4, UAS-Dp110*^{*D954A*}/*+;* EP(3)/+ progeny flies are compared to the eyes of at least five *ey-Gal4, UAS-Dp110*^{*D954A*}/+; balancer/+ progeny flies with the same sex. The same criterion is used to select potential positive EP(3) lines as in the case of EP(X) lines. Statistical analysis is used to verify the positive lines.

### Statistical analysis of the sizes of fly eyes

For each respective genotype, twenty fly eyes are photographed using a Zeiss dissecting microscope equipped with a Canon digital camera (Kodak Professional DCS 520, Eastman Kodak, , Rochester, NY). Images are processed by Photoshop 5.0 software (Adobe Systems Incorporated, , San Jose, California). To estimate the eye size ( two dimensional surface area based on photograph), an eye is approximated by an oval, thus both the long (a) and short (b) axis of an eye are measured. The eye size is calculated as ((a+b)/4)²π. The Student's t-Test (one-tailed, two-sample unequal variance) is used to test if there is statistically significant (p < 0.05) difference between the mean eye size of two genotypes. Before the t-Test is applied, the eye size data or its log transformation is tested for normality using the Kolmogorov-Smirnov test (Sokal, R.R. and Rohlf, F.J. (1981) Biometry. W.H.Freeman and Company, New York. 716pp.). SigmaStat for Windows Version 2.03 (SPSS, Inc. Chicago, IL) is used for statistical analysis of fly eye size. Because EP(X) insertion is on the X chromosome, the cross EP(X)/Y; +/+ x X/X; *ey-Gal4, UAS-Dp110*^{*D954A*}/*Balancer* produces the progeny with the genotype EP(X)/X ; *ey-Gal4, UAS-Dp110*^{*D954A*}/*+* but not the genotype X/X; *UAS-Dp110*^{*D954A*}/*+* needed for internal comparison. Thus, external X/X; *UAS-Dp110*^{*D954A*}/*+* flies are used (see Table 2).

### Genetic assay development

Two established genetic techniques are used to create the transgenic Drosophila disclosed herein. One is the binary Gal4/UAS system for tissue-specific transcription of a gene In this system, a fly strain expressing yeast transcription factor GAL4 in tissue X (often referred to as a Gal4 line or a Gal4 driver) is crossed to a fly strain carrying a transgene with GAL4 binding site UAS (upstream activation sequences) fused to gene Y (UAS-Y). Thus, in the progeny, gene Y is expressed specifically in tissue X.

The other genetic technique used is EP element mutagenesis (Rorth, P., et al. 1998 Development 125, 1049-57). The EP element is a synthetic, modified P element containing UAS sequences and a basal promoter at one end. The EP element can randomly insert into the *Drosophila* genome, often at the 5' end of a gene. Because the EP element is asymmetric with only one end containing the UAS sequence and a basal promoter, the effect of an EP insertion on nearby genes is influenced by the orientation of the inserted EP element. It has been shown that at the insertion site, the gene located proximal to the UAS and basal promoter-containing end of an EP element often has GAL4-dependent expression However, an EP insertion may also disrupt a cis element of the nearby gene and affect its expression in a Gal4-independent manner. A large collection of EP insertion strains are publicly available (see www.fruitfly.org).

### Real time PCR analysis of RNA levels

A N6 random primer (Roche Applied Science, Indianapolis, IN) is used for the reverse transcription reaction. The primer pairs and probe for each transcription unit is commercially available from Applied Biosystems (Foster City, CA). Real-time PCR is done using the ABI Prism 7700 Sequence Detection System according to the manufacturer's instruction (Applied Biosystems). Primer concentrations are 0.2 pmol/ul and the probe concentration is 0.1 uM, in a 25ul reaction. Cycles are 50°C for 2min, 95°C for 10min, followed by 40 cycles of 95°C for 15sec, 60°C for 1 min. Data is analyzed using the relative standard curve method as described in the User Bulletin#2 Manual for the ABI Prism 7700 Sequence Detection System (Applied Biosystems). All RNA levels are normalized by using the endogenous RNA levels of the ribosomal protein rp49 gene in each sample according to conventional methods. For X chromosome EP lines, EP/+ virgin females are crossed to hspGal4/CyO males. Male progenies of the genotype EP/Y; hspGal4/+ are collected and made to 8 vials with 10 flies per vial. 4 vials are kept at 25°C and 4 vials are subjected to 6 cycles of heat-shock and rest with each cycle made at 1 hr at 37°C and 3 hrs at 25°C. Using conventional methods, total RNAs are prepared from the 10 flies from each heat-shocked vial after heat shock and from unheat-shocked vials. Male progenies of the genotype +/Y; hspGal4/+, the genotype EP/Y; +/CyO, the genotype +/Y; +/CyO are similarly treated and total RNAs prepared. Thus for each transcription unit assayed of each EP line , the following data table is obtained (where hs refers to heat shock and nhs is no heat shock):

**Table 1A**

| Genotype | heat-shock | RNA levels of a transcription unit (mean value, normalized) |
|---|---|---|
| EP/Y;hspGal4/+ | nhs | A |
| EP/Y;hspGal4/+ | hs | A' |
| +/Y;hspGal4/+ | nhs | B |
| +/Y;hspGal4/+ | hs | B' |
| EP/Y; +/CyO | nhs | C |
| EP/Y; +/CyO | hs | C' |
| +/Y;+/CyO | nhs | D |
| +/Y;+/CyO | hs | D' |

The Student t-Test is used to determine if there is statistical difference between value A and A'. If there is a difference, it would indicate that there is a heat shock-dependent effect which may act through the interaction between Gal4 and EP. To correct for the independent effect of heat-shock alone, Gal4 alone and EP alone, the ratio of (A'/A)/((B'/B+C'/C+D'/D)/3) is determined and used to indicate the Gal4-dependent effect of EP on the transcription unit RNA level. (See Table 4, column "Gal4 effect"). The EP insertion may disrupt the function of a cis regulatory element of a nearby transcription unit at the insertion point. This insertion-effect is independent of Gal4. (See Table 4, column "Insert effect"). To determine this effect, Student t-Test is used to determine if there is a statistical difference between value C and D. If there is a difference, the ration of C/D is used to indicate the insertion-effect of a EP on a transcription unit RNA level.

The same method is used to analyze the second chromosome and third chromosome EP lines.

### Example 1

### Initial Genetic Screen

To generate an easily visible adult phenotype as a basis for genetic modifier screens to identify proteins involved in the *Drosophila* insulin signaling pathway, we sought to disrupt this pathway function in fly eyes. This is because (1) the eye is not essential for flies, (2) the eye phenotype is easily scored, and (3) reducing insulin signaling in the developing eyes causes a small eye phenotype (Leevers, SJ et al, EMBO J. 1996 Dec 2;15(23):6584-94).

For the small eye phenotype-based genetic modifier screen to work, we reasoned that the phenotype must be modifiable by changing the expression level of known insulin signaling genes. This is indeed what we observed. The small eye phenotype is suppressed by over-expression of *Drosophila* PKB via the *UAS-PKB* transgene, which acts downstream of PI3K. Conversely, the small eye phenotype is also enhanced by over-expression of PTEN, which acts antagonistically to PI3K (See also Goberdhan, D. C. et al.(1999) Genes Dev 13, 3244-58.; Huang, H., et al., Development 126, 5365-72.). These results demonstrate that we should be able to identify new genes in the insulin signaling pathway by a genetic modifier screen using the EP insertion strains, which often cause GAL4-dependent over-expression of a nearby gene. These genes can be either positively acting (suppressors of the small eye phenotype), or negatively acting (enhancers of the small eye phenotype) in insulin signaling.

We expressed a dominant-negative form of *Drosophila* PI3K catalytic subunit, Dp110^{D954A}, by using the *ey-Gal4* transgene which expresses GAL4 in the developing eyes, and the *UAS- Dp110*^{*D954A*}transgene (Hazelett, D. J., et al. (1998). Development 125, 3741-51. ); Leevers, SJ et al, EMBO J. 1996 Dec 2;15(23):6584-94).

As disclosed herein, flies carrying a chromosome with both the *ey-Gal4* and the *UAS-Dp110*^{*D954A*} transgenes have smaller eyes as compared to wild type control flies. This small eye phenotype has 100% penetrance, indicating strong inhibitory effect of the Dp110^{D954A} protein in the insulin pathway.

The *Drosophila* genome contains about 14,000 predicted genes (Adams et al., 2000 Science 287 2185-95). These genes are defined by the predicted transcripts ( sometimes referred to as transcription units or "Celera Transcripts" (CTs), see www.Celera.com). Based on the known functions and predicted functions, these genes or transcription units are classified into different functional categories or "bins". An analysis of publicly available EP strains revealed 1536 EP strains that have EP elements inserted within 10 Kb distance upstream from the start codon (ATG) of 483 genes (CTs). These genes belong to the functional bins of peptidase, protein kinases/phophatases, signal transduction molecules, transporters, ligand-binding proteins, transcription factors, enzymes and other types of molecules. The functional bin categories are based on the Celera *Drosophila* Jamboree Release 1.

As an initial screen, 112 EP strains that have an EP element inserted next to 68 predicted genes that encode protein kinases/phosphatases or their interacting factors are crossed to flies carrying the *ey-Gal4 UAS-Dp110*^{*D954A*} chromosome and eyes of adult progenies examined as described above. Three EP strains are found to be able to suppress the small eye phenotype induced by *Dp110*^{*D954A*}*-* EP(3)3553, EP(X)0382 and EP(3)3459 and are described below:

### EP(3)3553 /PDK

Statistical analysis confirms that EP(3)3553 is a suppressor of *Dp110*^{*D954A*}*.* The mean eye size of the XX; *ey-Gal4 UAS-Dp110*^{*D954A*} /+ flies is 8.39×10⁻² mm² while the mean eye size of their sisters XX; *ey-Gal4 UAS-Dp110*^{*D954A*} /+; EP(3)3553/+ flies is 1.19×10⁻¹ mm². The two means are significantly different (*p* < 0.001, Student's t-Test hereafter). The mean eye size of the XY; *ey-Gal4 UAS-Dp110*^{*D954A*} /+ flies is also smaller that that of their brothers XY; *ey-Gal4 UAS-Dp110*^{*D954A*} /+; EP(3)3553/+ flies (7.39×10⁻² mm² vs. 9.20×10⁻² mm², *p*<0.001). These results show that EP(3)3553 suppresses the small eye phenotype caused by *Dp110*^{*D954A*}*.* See Table 1 below.

**Table 1**

| **EP(3)3553 suppresses *Dp110***^{***D954A***} | | | | |
|---|---|---|---|---|
| | 1 | 2 | 3 | 4 |
| | Genotypes^{a} | Mean^{b} (eye size, mm²) | S.D.^{c} | n^{d} |
| 1 | XX; *ey-Ga14, UAS-Dp110*^{*D954A*}/*+;* EP(3)3553/+ | 1.19×10⁻¹ | 1.43×10⁻² | 20 |
| 2 | XX; *ey-Gal4, UAS-Dp110*^{*D954A*}/*+;* +/+ | 8.39×10⁻² | 1.60×10⁻² | 20 |
| 3 | XY*; ey-GaI4, UAS-Dp110*^{*D954A*}/*+;* EP(3)3553/+ | 9.20×10⁻² | 1.02×10⁻² | 20 |
| 4 | XY*; ey-Gal4, UAS-Dp110*^{*D954A*}/*+; +*/*+* | 7.39×10⁻² | 1.18×10⁻² | 20 |

| | | | | |
|---|---|---|---|---|
| a. The abbreviated progeny genotypes of the cross X/Y*;* +/+*;* EP(3)3553/*TM6B,Tb* x X/X*; ey-Gal4, UAS-Dp110*^{*D954A*}/*CyO; +*/*+. TM6B* and *CyO* are balancer chromosomes ( se methods section, above). b. Student's t-Test shows that the means of Row 1 and Row 2 differ at the significant level with p< 0.001. The means of Row 3 and Row 4 differ at the significant level with p< 0.001. c. S.D. represents standard deviation. d. The total number of eyes measured. | | | | |

EP(3)3553 has an EP element inserted on the third chromosome at the 5' end noncoding sequences of the *Drosophila* gene *Pk61C,* which is the fly ortholog of mammalian PDK1, Genbank accession number NM_002613 (http://flybase.bio.indiana.edu.). PK61C is the only gene found within 10 Kb region on both sides of the EP(3)3553 insertion site. The relative position of the *Pk61C* gene to the EP(3)3553 element is such that the 5' end of *Pk61C* is proximal to the UAS-containing end of the EP(3)3553 element (http://fiybase.bio.indiana.edu). Thus, it is expected that GAL4 would induce the expression of *Drosophila* PDK1. Since PDK1 is known to act downstream of PI3K in the insulin signaling pathway, it is also predicted that over-expression of PDK1 might suppress *Dp110*^{*D594A*}-induced phenotypes. Thus, given the involvement of PDK1 in the insulin signaling pathway, the identification of EP(3)3553 as a suppressor of *Dp110*^{*D594A*}-induced small eye phenotype strongly suggests that our genetic screen can be used to identify molecules relevant to the insulin pathway.

### EP(X)0382 / SHP-2

Statistical analysis also confirms that EP(X)0382 significantly suppresses the small eye phenotype induced by over-expression of *Dp110*^{*D954A*}*.* The mean eye size of EP(X)0382/X; *ey-Gal4, UAS-Dp110*^{*D954A*}/*+* flies (1.04×10⁻¹ mm²) is significantly larger than that of the XX; *ey-Gal4, UAS-Dp110*^{*D954A*}/*+* flies (9.05×10⁻² mm²; *p* < 0.0001), indicating that EP(X)0382 suppresses the function of *Dp110*^{*D954A*}*.* See Table 2 below:

**Table 2**

| **EP(X)0382 suppresses *Dp110***^{***D954A***} | | | | | |
|---|---|---|---|---|---|
| | 1 | 2 | 3 | 4 | 5 |
| | Genotypes^{a} | Mean (eye size, mm²) | Mean^{b} (corrected eye size, mm²) | S.D.^{c} | n |
| 1 | XX; *ey-Gal4, UAS-Dp110*^{*D954A*}/*+* | 9.05x10⁻² | 9.05×10⁻² | 1.06×10⁻² | 20 |
| 2 | XY; *ey-Gal4, UAS-Dp110*^{*D954A*}/*+* | 7.24x10⁻² | 7.24×10⁻² | 1.04×10⁻² | 20 |
| 3 | EP(X)0382/X*; ey-Gal4, UAS- Dp110*^{*D954A*}/*+* | 1.06×10⁻¹ | 1.04×10⁻¹ | 9.72x10⁻³ | 20 |
| 4 | XY*; ey-Gal4, UAS-Dp110*^{*D954A*}/*+* | 7.38×10⁻² | 7.24×10⁻² | 9.87×10⁻³ | 20 |

| | | | | | |
|---|---|---|---|---|---|
| a. Row 1 and 2 contain the abbreviated genotypes of the stock *w*/*Y; ey-GaI4, UAS-Dp110*^{*D954A*}/*CyO.* Rows 3-4 contain the abbreviated progeny genotypes of the cross EP(X)0382/Y; +/+ x *wlw; ey-GaI4, UAS-Dp110*^{*D954A*}/*CyO. CyO* is a balancer chromosome (see methods section, above). b. Flies in Rows 1-2 are siblings. Flies in Rows 3-4 are siblings. But flies in Rows 1-2 are not siblings of flies in Rows 3-4. Therefore, they could have experienced somewhat different growth conditions which affect the body size including eye size. Thus for the eye size comparisons between them to be meaningful, the measured eye sizes of Rows 3-4 are corrected, based on the assumption that the flies of the common genotype XY; *ey-Gal4, UAS-Dp110*^{*D954A*}/*+* (Row 2, Column 1; Row 4, Column 1) should have the same mean eye size. Each of the 20 eyes measured in Rows 3-4 are corrected by the factor 0.981 (7.24×10⁻²/7.38×10⁻², see Column 2). This column shows the average of the corrected eye sizes. Student's t-Test shows that the means of Row 1 and Row 3 differ at the significant level with p< 0.001. c. The standard deviation is based on the corrected eye sizes. | | | | | |

EP(X)0382 strain has an EP element inserted on the X chromosome at the 5' end of the *Drosophila* gene *corkscrew (csw),* which is the fly ortholog of the human protein-tyrosine phosphatase *SHP-2* gene (GenBank accession number NM_002834). The insertion site is 201 nucleotides upstream of the start site of a *csw cDNA* (GenBank Accession U19909; www.fruitfly.org). The relative position of the *csw* gene to the EP(X)0382 element is such that the 5' end of *csw* is proximal to the non-UAS-containing end of the EP(X)0382 element. There is no other gene found within the 10 Kb region on the other side of the EP(X)0382 insertion site. Using quantitative real-time RT-PCR,no effect of the EP on csw RNA level was detectable at the level of analysis.

*SHP-2* and *csw* have been shown to play a positive role in receptor tyrosine kinase signaling pathways, such as those involving the PDGF receptor and the EDGP receptor (Allard et al., (1996. Development 122, 1137-46; Bennett, A. M., et al. (1994). Proc Natl Acad Sci U S A 91, 7335-9; Feng, G. S. et al. (1993) Science 259, 1607-11; Perkins, L. A., et al., (1996). Dev Biol 180, 63-81; Vogel, W., et al. 1993 Science 259, 1611-4; .Xiao, S., et al. (1994) J Biol Chem 269, 21244-8).

*SHP-2* has also been implicated in insulin signaling, although whether it plays a positive or negative role is presently unknown ( Kharitonenkov, A. et al., (1995). J Biol Chem 270, 29189-93; Maegawa, H., et al.(1999) J Biol Chem 274, 30236-43.; Myers, M. G., et al. (1998) J Biol Chem 273, 26908-14; Ugi, S., et al. J Biol Chem 271, 12595-602).

Since *SHP-2* is a known gene in the mammalian insulin signaling pathway, it suggests that the modifier screen disclosed herein may be used to identify molecules relevant to human diabetes.

### EP(3)3459/ KIAA0336

The suppression of the *Dp110*^{*D954A*}-induced small eye phenotype by EP(3)3459 is statistically significant. In both XX and XY flies, the presence of EP(3)3459 insertion increases the mean eye size of *ey-Gal4, UAS-Dp110*^{*D954A*}/*+* flies (9.71 ×10⁻² mm² versus 9.09×10⁻² mm² in XX flies, p<0.05; 8.10×10⁻² mm² versus 7.13×10⁻² mm² in XY flies, p<0.003). See Table 3 below.

**Table 3**

| **EP(3)3459 suppresses *Dp110***^{***D954A***} | | | | |
|---|---|---|---|---|
| | 1 | 2 3 4 | | |
| | Genotypes^{a} | Mean^{b} (eye size, mm²) | S.D. | n |
| 1 | XX*; ey-Gal4, UAS-Dp110*^{*D954A*}/*+;* EP(3)3459/+ | 9.71×10⁻² | 1.14×10⁻² | 20 |
| 2 | XX*; ey-Gal4, UAS-Dp110*^{*D954A*}/*+; +*/*+* | 9.09×10⁻² | 1.10×10⁻² | 20 |
| 3 | XY; *ey-Gal4, UAS-Dp110*^{*D954A*}/*+;* EP(3)3459/+ | 8.10×10⁻² | 8.75×10⁻³ | 20 |
| 4 | XY*; ey-Gal4, UAS-Dp110*^{*D954A*}/*+;* +/+ | 7.13×10⁻² | 1.03×10⁻² | 20 |

| | | | | |
|---|---|---|---|---|
| a. The abbreviated progeny genotypes of the cross X/Y; +/+; EP(3)3459/*TM6B,Sb,Tb* × X/X; *ey-GaI4, UAS-Dp110*^{*D954A*}/*CyO; +*/*+. TM6B* and *CyO* are balancer chromosomes (see methods section above) b. Student's t-Test shows that the means of Row 1 and Row 2 differ at the significant level with p< 0.05. The means of Row 3 and Row 4 differ at the significant level with p< 0.003. | | | | |

EP(3)3459 strain has an EP element inserted on the third chromosome at about 5 Kb upstream from the 5' end of the *Drosophila casein kinase II-alpha subunit interactor-3 (Ckll-α-i3,* Genbank accession number AF090440). The 5' end of *Ckll-α-i3* is proximal to the non-UAS-containing end of the EP(3)3459 element. On the other side of the EP(3)3459 insertion site, no gene was found within the 10 Kb region. Quantitative RT-PCR (Taqman) experiments indicate that this EP element causes 4 fold loss-of-expression of *Ckll-α-i3.*

The human homolog of *Ckll-α-i3* is suspected to be the human *KIAA0336* gene (GenBank accession number AB002334) Nagase, T., et al. (1997) DNA Res 4, 141-50.

The *KIAA0336* gene encodes a protein with 1583 amino acid residues with no known function. The cDNA that defines *KIAA0336* was derived from brain tissue. The *KIAA0336* gene is expressed in all tissue categories except the stomatognathic system based on LifeSeq®Gold 5.1. It has the highest expression in the hemic and immune system, as judged by the percentage of *KIAA0336* gene-derived cDNA clones among the total number of sequenced cDNA clones from the hemic and immune system. The apparent lack of the *KIAA0336* gene-derived cDNA clones in the stomatognathic system libraries may be due to the lower number of sequenced cDNA clones (10,988) from the libraries of the stomatognathic system as compared to 648,215 sequenced clones from the libraries of the hemic and immune system.

*CkII-α-i3* was identified through a two-hybrid interaction with CKII *α* subunit (see also the Genbank annotation, Genbank accession number AF090440). It encodes a putative nuclear protein with CKII consensus phosphorylation sites. While the function of *Ckll-α-i3* has not been reported, CKII is likely involved in the insulin signaling pathway based on data which shows that a CKII inhibitor blocks the insulin-induced DNA-binding activity of nuclear extracts and the CKII α subunit interacts with the insulin receptor substrate-1 (IRS-1) in vitro ( Kim, S. J., and Kahn, C. R. (1997) Biochem J 323, 621-7.; Li et al., 1999). It is contemplated herein that the human ortholog of *Ckll-a-i3 ,* KIAA0336, plays a role in the insulin signaling pathway, and as such is a suitable drug target for the development of therapeutics to treat, prevent or ameilorate pathological conditions associated with the dysregulation of the insulin signaling pathway.

Thus, we have expressed a dominant negative form of the *Drosophila* phosphoinositide 3-kinase (PI3K) in developing eye. This results in a small eye phenotype in adult flies. The small eye phenotype is suppressed by over-expression of protein kinase B (PKB), which acts downstream of PI3K in the insulin signaling pathway (Lasko, P. Clin Genet. 2002 Nov;62(5):358-67), suggesting that the small eye phenotype can be used as an assay for a genetic modifier screen. Our studies identified three mutations that suppress the small eye phenotype. One mutation is linked to the *Drosophila* ortholog of the *3'-phosphoinositide-dependent kinase 1* gene *(PDK1).* Another mutation is linked to the *Drosophila* ortholog of the protein-tyrosine phosphatase *SHP-2* gene. Both *PDK1* and *SHP-2* are known genes in the mammalian insulin signaling pathway, suggesting that the screen will identify molecules relevant to human diabetes. The third mutation is linked to the *Drosophila* gene *Ckll-α-i3,* whose human ortholog is *KIAA0336.*

### Example 2

### Identification of Additional Proteins

Using methods described above, additional proteins were identified which are suspected to play a role in the insulin signaling pathway. These genes and their human homologs (listed in column "best hu") (as well as KIAA0336, discussed above) are set forth below in Table 4. The genetic screen identified the suppressors, which were divided into categories depending on strength of suppression, where S1 is the strongest and S4 the weakest.

**Table 4 Modifiers**

| **Gene** | **EP** | **females** | **males** | **Celera transcript** | **Gal4- effect** | **insert-effect** | **best hu** | **Best hu blastp E value** |
|---|---|---|---|---|---|---|---|---|
| FMR2 protein | EP(2)2172 | S1 | S1 | CG8817(lilli) | no | 0.53x | NM_002025 | 1.E-14 |
| nucleoside diphosphatase (ER-UDPase) | EP(2)2172 | S1 | S1 | CT10292 | 2.2x | 0.79x | | |
| inositol hexkisphosphate kinase 3 | EP(2)2440 | S1 | S1 | CT28369 | 2.7x | 0.60x | NM_054111 | 5.E-39 |
| RNA polymerase I 16Kd subunit | EP(2)2483 | S2 | S1 | CT29944/CG1068 5 | 70x | 1.34x | | |
| brain tumor | EP(2)2483 | S2 | S1 | CG10719 | | | NM_033279.1 | 5.E-37 |
| neuroligin 3 | EP(2)2615 | S1 | S1 | CT12983 | 5.5x | no | NM_018977 | 7.E-68 |
| retinoblastoma-binding protein 5 | EP(3)3121 | S1 | S1 | CT17654 | 35x | 1.9x | NM 005057.1 | 0.E+00 |
| polypeptide chain release factor 1 | EP(3)3121 | S1 | S1 | CT17696 | no | 0.78x | | |
| CYSTEINE STRING PROTEIN (CSP) | EP(3)3141 | S1 | S2 | CT19114 | no | 0.5x | S70516 | 7.E-40 |
| hypothetical protein | EP(3)3141 | S1 | S2 | CT36401 | 13x | no | NM_016472 | 6.E-10 |
| TRANSCRIPTION FACTOR ADF-1 (ADH | EP(3)3354 | S1 | S1 | CT16251 | 36x | no | none | |
| DISTAL FACTOR 1) | | | | | | | | |
| membrane protein TMS-2 | EP(3)3355 | S1 | S1 | CT15063 | 7x | no | AB033079.1 | 6.E-90 |
| unknown | EP(3)3355 | S1 | S1 | CT14836(failed axon connection) | no | 0.05x | XM_046613.1 | 4.E-22 |
| 3-phosphoinositide dependent protein | EP(3)3553 | S1 | S1 | CT42509 | 140x | | | |
| kinase-1 | | | | | | | | |
| hypothetical protein | EP(3)3628 | S1 | S1 | CT5336 | 10x | no | NM 015343.1 | 6. E-37 |
| SH3 domain-containing protein SH3d19 | EP(3)3634 | S1 | S1 | CT22037 | 2.4x | no | XM_037453 | 1.E-12 |
| poly (rC)-binding protein 3 | EP(3)3638 | S1 | S2 | contig_4403_1 | | | NM_020528.1 | 1.E-76 |
| nucleolar RNA-assodated protein alpha | EP(3)3652 | S1 | S2 | CT37030 | | | NM_022917 | 1.E-98 |
| Casein kinase I, gamma 3 | EP(3)3652 | S1 | S2 | gish | | | NM 004384.1 | 1.E-170 |
| acyl-Coenzyme A dehydrogenase, | EP(3)3660 | S2 | S1 | CT12987 | no | 0.60x | NM_001609 | 1.E-142 |
| short/branched chain precursor | | | | | | | | |
| (ACADSB) | | | | | | | | |
| ribosomal protein L26 | EP(3)3660 | S2 | S1 | CT21207 | 1.6x | no | | |
| myosin phosphatase target subunit 1 | EP(3)3723 | S1 | S1 | CT18415 | 4.5x | no | NM_002480 | 8.E-73 |
| GPI transamidase | EP(X)0427 | S1 | S1 | CT14360 | 24x | 1.6x | XM_039644 | 1.E-118 |
| no good hu/mo homologs | EP(X)0427 | S1 | S1 | CT14236 | no | no | | |
| mouse preprocathepsin B | EP(X)1101 | S2 | S1 | CT30795 | no | 0.54x | NM 001908.1 | 1.E-106 |
| no good hu/mo homologs | EP(X)1101 | S2 | S1 | CT31077 | 7.8x | no | | |
| tousled-like kinase 2/SNARE protein | EP(X)1413 | S1 | S1 | CG2829/CG12462 | | | NM_006852.1 | 1.E-146 |
| kinase SNAK | | | | | | | | |
| CKII-alpha-i3 (KIAA0336) | EP(3)3459 | S5 | S5 | CG3217 | no | 0.26x | AB002334 | 2.3E-6 |

To verify that a protein (gene listed in Table 4 ) (i.e. "a modifier gene") is involved in insulin signaling and metabolism, one may examine if the perturbation of a modifier gene will cause a change in the expression level of an insulin responsive sequence (IRS)-controlled reporter gene. It is known that the activation of the insulin signaling pathway leads to the inhibition of the forkhead transcription factors that bind to the insulin responsive sequences. Thus, changes in the insulin signaling level by a modifier may lead to changes in the expression level of the IRS-controlled genes. In addition, because the insulin signaling pathway controls glucose and lipid metabolism, one may examine if the perturbation of a modifier gene causes a change in the glycogen or lipid level in the fly.

The genetic screen is designed to identify both genes that activate and the genes that repress the insulin signaling pathway. However, because stimulating the insulin signaling pathway is needed for the treatment of diabetes, the genes that normally inhibit insulin signaling may be the preferred drug targets.

### Example 3

### Additional Modifier Screens

In addition to the genetic screen discussed above which is based on the dominant phenotype induced by a dominant-negative form of fly PI3K, new genetic screening methods may also be developed using other known genes in the insulin signaling pathway. For example, over-expression of PTEN in developing eyes also causes a small eye phenotype. Based on this phenotype, a suppressor/enhancer screen can be established. We have seen that over-expression of a dominant active PI3K causes lethality in flies, and this lethality can be suppressed by overexpression of PTEN (unpublished observation). Thus a "suppression of lethality-based screen" could also be used to identify new genes with similar properties of PTEN in the insulin pathway. This kind of screen is especially desirable since it is a "selection"-based screen. Furthermore, a fluorescent reporter-based genetic screen may also be used. The advantage of this approach is that it is adaptable for high throughput automation. For example, it is known that the insulin pathway controls the activity of the FKHR-type forkhead transcription factors. A fluorescent reporter can be made using the forkhead binding sites to control flourescent marker expression. Flies carrying this reporter screened with a fluorescent *Drosophila* larvae sorter would be an efficient system to identify new genes in the insulin pathway.

## Claims

1. A method to treat, prevent or ameliorate pathological conditions associated with dysregulation of the insulin signaling pathway comprising administering to a subject in need thereof an effective amount of a modulator of a protein selected from the group consisting of those disclosed in Table 4.

2. The method of claim 1 wherein said condition is Type II diabetes.

3. The method of claim 1 wherein said condition is the Type A syndrome of insulin resistance.

4. The method of claim 1 wherein said modulator inhibits the biochemical function of said protein in said subject.

5. The method of claim 4 wherein said modulator comprises one or more antibodies to said protein, or fragments thereof, wherein said antibodies or fragments thereof can inhibit the biochemical function of said protein in said subject.

6. The method of claim 1 wherein said modulator enhances the biochemical function of said protein in said subject.

7. The method of claim 1 wherein said modulator inhibits gene expression of said protein in said subject.

8. The method of claim 7 wherein said modulator comprises any one or more substances selected from the group consisting of antisense oligonucleotides, triple helix DNA, ribozymes, RNA aptamers, siRNA, double stranded RNA and single stranded RNA wherein said substances are designed to inhibit gene expression of said protein.

9. The method of claim 1 wherein said modulator enhances the gene expression of said protein in said subject.

10. A method to treat, prevent or ameliorate pathological conditions associated with dysregulation of the insulin signaling pathway comprising administering to a subject in need thereof a pharmaceutical composition comprising an effective amount of a modulator of a protein selected from the group consisting of those disclosed in Table 4.

11. The method of claim 10 wherein said condition is Type II diabetes.

12. The method of claim 10 wherein said condition is the Type A syndrome of insulin resistance.

13. The method of claim 10 wherein said modulator inhibits the biochemical function of said protein in said subject.

14. The method of claim 13 wherein said modulator comprises one or more antibodies to said protein, or fragments thereof, wherein said antibodies or fragments thereof can inhibit the biochemical function of said protein.

15. The method of claim 10 wherein said modulator enhances the biochemical function of said protein in said subject

16. The method of claim 10 wherein said modulator inhibits gene expression of said protein in said subject.

17. The method of claim 16 wherein said modulator comprises any one or more substances selected from the group consisting of antisense oligonucleotides, triple helix DNA, ribozymes, RNA aptamers, siRNA, double stranded RNA and single stranded RNA wherein said substances are designed to inhibit gene expression of said protein.

18. The method of claim 10 wherein said modulator enhances gene expression of said protein in said subject.

19. A method to identify modulators useful to treat, prevent or ameliorate pathological conditions associated with dysregulation of the insulin signaling pathway comprising assaying for the ability of a candidate modulator to modulate the biochemical function of a protein selected from the group consisting of those disclosed in Table 4.

20. The method of claim 19 wherein said method further comprises assaying for the ability of an identified modulator to reverse the pathological effects observed in animal models of said conditions.

21. The method of claim 19 wherein said method further comprises assaying for the ability of an identified modulator to reverse the pathological effects observed in clinical studies with subjects with said conditions.

22. The method according to claim 19 wherein said condition is Type II diabetes.

23. The method according to claim 19 wherein said condition is the Type A syndrome of insulin resistance.

24. A method to identify modulators useful to treat, prevent or ameliorate pathological conditions associated with dysregulation of the insulin signaling pathway comprising assaying for the ability of a candidate modulator to modulate gene expression of a protein selected from the group consisting of those disclosed in Table 4.

25. The method according to claim 24 wherein said method further comprises assaying for the ability of an identified inhibitory modulator to reverse the pathological effects observed in animal models of said condition.

26. The method according to claim 24 wherein said method further comprises assaying for the ability of an identified inhibitory modulator to reverse the pathological effects observed in clinical studies with subjects with said condition.

27. The method according to claim 24 wherein said condition is Type II diabetes.

28. The method according to claim 24 wherein said condition is the Type A syndrome of insulin resistance.

29. A pharmaceutical composition comprising a modulator to a protein selected from the group consisting of those disclosed in Table 4 in an amount effective to treat, prevent or ameliorate pathological conditions associated with dysregulation of the insulin signaling pathway in a subject in need thereof.

30. The pharmaceutical composition according to claim 29 wherein said condition is Type II diabetes.

31. The pharmaceutical composition according to claim 29 wherein said condition is the Type A syndrome of insulin resistance.

32. The pharmaceutical composition according to claim 29 wherein said modulator inhibits the biochemical function of said protein.

33. The pharmaceutical composition of claim 29 wherein said modulator comprises one or more antibodies to said protein, or fragments thereof, wherein said antibodies or fragments thereof can inhibit the biochemical function of said protein.

34. The pharmaceutical composition according to claim 29 wherein said modulator enhances the biochemical function of said protein.

35. The pharmaceutical composition according to claim 29 wherein said modulator inhibits gene expression of said protein.

36. The pharmaceutical composition of claim 29 wherein said modulator comprises any one or more substances selected from the group consisting of antisense oligonucleotides, triple helix DNA, ribozymes, RNA aptamer, siRNA, double stranded RNA and single stranded RNA wherein said substances are designed to inhibit gene expression of said protein.

37. The pharmaceutical composition according to claim 25 wherein said modulator enhances gene expression.of said protein.

38. A method to diagnose subjects suffering from pathological conditions associated with dysregulation of the insulin signaling pathway who may be suitable candidates for treatment with modulators to a protein selected from the group consisting of those disclosed in Table 4 comprising assaying mRNA levels of any one or more of said proteins in a biological sample from said subject wherein subjects with altered levels compared to controls would be suitable candidates for modulator treatment.

39. A method to diagnose subjects suffering from pathological conditions associated with dysregulation of the insulin signaling pathway who may be suitable candidates for treatment with modulators to a protein selected from the group consisting of those disclosed in Table 4 comprising detecting levels of any one or more of said proteins in a biological sample from said subject wherein subjects with altered levels compared to controls would be suitable candidates for modulator treatment.

40. A method to treat, prevent or ameliorate a pathological condition associated with dysregulation of the insulin signaling pathway comprising:
(a) assaying for mRNA levels of a protein selected from the group consisting of those disclosed in Table 4 in a subject; and,
(b) administering to a subject with altered levels of mRNA of said protein compared to controls a modulator to said protein in an amount sufficient to treat, prevent or ameliorate the pathological effects of said condition.

41. The method of claim 40 wherein said condition is Type II diabetes.

42. The method of claim 40 wherein said condition is the Type A syndrome of insulin resistance.

43. The method of claim 40 wherein said modulator enhances the gene expression of said protein.

44. The method of claim 40 wherein said modulator inhibits the gene expression of said protein.

45. A method to treat, prevent or ameliorate a pathological condition associated with dysregulation of the insulin signaling pathway comprising:
(a) assaying for levels of a protein selected from the group consisting of those disclosed in Table 4 in a subject; and,
(b) administering to a subject with altered levels of said protein compared to controls a modulator to said protein in an amount sufficient to treat, prevent or ameliorate the pathological effects of said condition.

46. The method of claim 45 wherein said condition is Type II diabetes.

47. The method of claim 45 wherein said condition is the Type A syndrome of insulin resistance.

48. The method of claim 45 wherein said modulator enhances the biochemical function of said protein.

49. The method of claim 45 wherein said modulator inhibits the biochemical function of said protein.

50. A diagnostic kit for detecting mRNA levels of a protein selected from the group consisting of those disclosed in Table 4 in a biological sample, said kit comprising:
(a) a polynucleotide of a polypeptide set forth in Table 4 or a fragment thereof;
(b) a nucleotide sequence complementary to that of (a);
(c) a polypeptide of Table 4 of the present invention encoded by the polynucleotide of (a),
(d) an antibody to the polypeptide of (c)
(e) an RNAi sequence complementary to that of (a)
wherein components (a), (b), (c), (d) or (e may comprise a substantial component.

51. A diagnostic kit for detecting levels of a protein selected from the group consisting of those disclosed in Table 4 in a biological sample, said kit comprising:
(a) a polynucleotide of a polypeptide set forth in Table 4 or a fragment thereof;
(b) a nucleotide sequence complementary to that of (a);
(c) a polypeptide of Table 4 of the present invention encoded by the polynucleotide of (a),
(d) an antibody to the polypeptide of (c)
(e) an RNAi sequence complementary to that of (a)
wherein components (a), (b), (c), (d) or (e) may comprise a substantial component.

52. A method to identify genetic modifiers of the insulin signaling pathway, said method comprising
(a) providing a transgenic fly whose genome comprises a DNA sequence encoding a polypeptide comprising *Dp110*^{*D954A*}*.,* said DNA sequence operably linked to a tissue specific control sequence, and expressing said DNA sequence, wherein expression of said DNA sequence results in said fly displaying a transgenic phenotype;
(b) crossing said transgenic fly with a fly containing a mutation in a known or predicted gene; and
(c) screening progeny of said crosses for flies that carry said DNA sequence and said mutation and display modified expression of the transgenic phenotype as compared to controls.

53. The method of claim 52 wherein said DNA sequence encodes *Dp110*^{*D954A*}*.* and wherein said tissue specific expression control sequence comprises the eye specific enhancer, ey-Gal4.

54. The method of claim 53 wherein expression of said DNA sequence results in said fly displaying the "small eye" phenotype.

55. A method to identify targets for the development of therapeutics to treat, prevent or ameliorate pathological conditions associated with dysregulation of the insulin signaling pathway said method comprising identifying the human homologs of the genetic modifiers identified according to the method of claim 52.

56. The method of claim 55 wherein said condition is Type II diabetes.

57. The method of claim 55 wherein said condition is the Type A syndrome of insulin resistance.
